# EUROPÄISCHE PATENTANMELDUNG

(11) **EP 3 804 615 A1**
(43) Veröffentlichungstag der Anmeldung: **14.04.2021**
(21) Anmeldenummer: 19201937.0
(22) Anmeldetag: 08.10.2019
(51) Int. Cl.: A61B 5/055, A61B 5/00, G01R 33/56

(54) **ERZEUGUNG VON MRT-AUFNAHMEN DER LEBER**

(71) Anmelder: Bayer AG, 51373 Leverkusen (DE)
(72) Erfinder: Knobloch, Gesine, 10437 Berlin (DE); Lienerth, Christian, 60486 Frankfurt/Main (DE); Rohrer, Martin, 12203 Berlin (DE); Uber, Arthur, 15208 Pittburgh (US)
(74) Vertreter: BIP Patents

(57) **Zusammenfassung**

Die vorliegende Erfindung befasst sich mit der Erzeugung von künstlichen MRT-Aufnahmen der Leber. Gegenstände der vorliegenden Erfindung sind ein Verfahren, ein System und ein Computerprogrammprodukt zur Erzeugung von MRT-Aufnahmen der Leber.

## Beschreibung

Die vorliegende Erfindung befasst sich mit der Erzeugung von künstlichen MRT-Aufnahmen der Leber. Gegenstände der vorliegenden Erfindung sind ein Verfahren, ein System und ein Computerprogrammprodukt zur Erzeugung von MRT-Aufnahmen der Leber.

Die Magnetresonanztomographie, abgekürzt MRT oder MR (engl. MRI: *Magnetic Resonance Imaging*), ist ein bildgebendes Verfahren, das vor allem in der medizinischen Diagnostik zur Darstellung von Struktur und Funktion der Gewebe und Organe im menschlichen oder tierischen Körper eingesetzt wird.

Bei der MR-Bildgebung werden die magnetischen Momente von Protonen in einem Untersuchungsobjekt in einem Grundmagnetfeld ausgerichtet, so dass sich eine makroskopische Magnetisierung entlang einer Längsrichtung einstellt. Diese wird anschließend durch das Einstrahlen von Hochfrequenz(HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten und ortsaufgelösten MR-Daten liegen zunächst als Rohdaten in einem Ortsfrequenzraum vor, und können durch anschließende FourierTransformation in den Ortsraum (Bildraum) transformiert werden.

Bei der nativen MRT werden die Gewebs-Kontraste durch die unterschiedlichen Relaxationszeiten (T1 und T2) und die Protonendichte erzeugt.

Die T1-Relaxation beschreibt den Übergang der Längsmagnetisierung (longitudinale Magnetisierung) in ihren Gleichgewichtszustand, wobei T1 diejenige Zeit ist, die benötigt wird, um 63,21% der Gleichgewichtsmagnetisierung vor der Resonanzanregung zu erreichen. Sie wird auch longitudinale Relaxaktionszeit oder Spin-Gitter-Relaxationszeit genannt.

Die T2-Relaxation beschreibt in analoger Weise den Übergang der Transversalmagnetisierung in ihren Gleichgewichtszustand.

MR-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren.

Superparamagnetische führen zu einer überwiegenden T2-Verkürzung, während paramagnetische Kontrastmittel im Wesentlichen zu einer Tl-Verkürzung führen. Eine Verkürzung der Tl-Zeit führt zu einer Zunahme der Signalintensität in T1-gewichteten Sequenzen, eine Verkürzung der T2-Zeit führt zu einer Abnahme der Signalintensität in T2-gewichteten Sequenzen.

Die Wirkung dieser Kontrastmittel ist indirekt, da das Kontrastmittel selbst kein Signal abgibt, sondern nur die Signalintensität der Wasserstoffprotonen in seiner Umgebung beeinflusst.

In T1-gewichteten Aufnahmen führen die paramagnetischen Kontrastmittel zu einer helleren (signalreicheren) Darstellung der Bereiche, die Kontrastmittel beinhalten, gegenüber den Bereichen, die kein Kontrastmittel beinhalten.

In T2-gewichteten Aufnahmen führen superparamagnetisches Kontrastmittel zu einer dunkleren (signalärmeren) Darstellung der Bereiche, die Kontrastmittel beinhalten, gegenüber den Bereichen, die kein Kontrastmittel beinhalten.

Sowohl eine signalreichere als auch eine signalärmere Darstellung führen zu einer Kontrastverstärkung.

Ein Beispiel für ein superparamagnetisches Kontrastmittel sind Eisenoxidnanopartikel (SPIO, engl.: *superparamagnetic iron oxide*).

Beispiele für paramagnetische Kontrastmittel sind Gadolinium-Chelate wie Gadopentetat-Dimeglumin (Handelsname: Magnevist® u.a.), Gadobenat-Dimeglumin (Handelsname: Multihance®), Gadotersäure (Dotarem®, Dotagita®, Cyclolux®), Gadodiamid (Omniscan®), Gadoteridol (ProHance®) und Gadobutrol (Gadovist®).

Nach ihrem Verteilungsmuster im Gewebe können extrazelluläre, intrazelluläre und intravaskuläre Kontrastmittel unterschieden werden.

Kontrastmittel auf der Basis der Gadoxetsäure zeichnen sich dadurch aus, dass sie spezifisch von Leberzellen, den Hepatozyten, aufgenommen werden, sich im Funktionsgewebe (Parenchym) anreichern und die Kontraste in gesundem Lebergewebe verstärken. Die Zellen von Zysten, Metastasen und der meisten Leberzellkarzinome arbeiten nicht mehr wie normale Leberzellen, nehmen das Kontrastmittel nicht oder kaum auf, werden nicht verstärkt dargestellt und werden damit erkennbar und lokalisierbar.

Beispiele für Kontrastmittel auf der Basis der Gadoxetsäure sind in US 6,039,931A beschrieben; sie sind kommerziell zum Beispiel unter den Markennamen Primovist® oder Eovist® erhältlich.

Der kontrastverstärkende Effekt von Primovist®/Eovist® wird durch den stabilen Gadoliniumkomplex Gd-EOB-DTPA (Gadolinium-Ethoxybenzyl-Diethylentriamin-Pentaessigsäure) vermittelt. DTPA bildet mit dem paramagnetischen Gadoliniumion einen Komplex, der eine extrem hohe thermodynamische Stabilität aufweist. Der Ethoxybenzylrest (EOB) ist der Vermittler der hepatobiliären Aufnahme des Kontrastmittels. Primovist® wird intravenös als Bolus, gewichtsadaptiert dosiert injiziert. Die empfohlene Dosis beträgt 0,025 mmol/kg Körpergewicht bzw. 0,1 ml/kg Körpergewicht.

Primovist® kann zur Detektion und Charakterisierung von Tumoren in der Leber eingesetzt werden. Die Blutversorgung des gesunden Lebergewebes erfolgt in erster Linie über die Pfortader (*Vena portae*), während die Leberarterie (*Arteria hepatica*) die meisten Primärtumoren versorgt. Nach intravenöser Bolusinjektion eines Kontrastmittels lässt sich dementsprechend eine Zeitverzögerung zwischen der Signalanhebung des gesunden Leberparenchyms und des Tumors beobachten.

Bei der durch Primovist® erzielten Kontrastverstärkung während der Anflutungsphase beobachtet man typische Perfusionsmuster, die Informationen für die Charakterisierung der Läsionen liefern. Die Darstellung der Vaskularisierung hilft, die Läsionstypen zu charakterisieren und den räumlichen Zusammenhang zwischen Tumor und Blutgefäßen zu bestimmen.

Bei T1-gewichteten Bildern führt Primovist® 10-20 Minuten nach der Injektion (in der hepatobiliären Phase) zu einer deutlichen Signalverstärkung im gesunden Leberparenchym, während Läsionen, die keine oder nur wenige Hepatozyten enthalten, z.B. Metastasen oder mittelgradig bis schlecht differenzierte hepatozelluläre Karzinome (HCCs), als dunklere Bereiche auftreten.

Primovist® ist gut verträglich. Die am häufigsten beobachteten Nebenwirkungen (≥ 0,5%) bei Patienten, die Primovist® erhalten sind Übelkeit, Kopfschmerzen, Hitzegefühl, Blutdruckanstieg, Rückenschmerzen und Schwindel. Es war eine Aufgabe der vorliegenden Erfindung die Anwendung von Primovist® insbesondere unter dem Gesichtspunkt der Patientenverträglichkeit weiter zu optimieren.

Diesem Anliegen widmet sich die vorliegende Erfindung mit den Gegenständen der unabhängigen Patentansprüche. Bevorzugte Ausführungsformen der vorliegenden Erfindung finden sich in den abhängigen Patentansprüchen, in dieser Beschreibung und in den Zeichnungen.

Ein erster Gegenstand der vorliegenden Erfindung ist ein Verfahren umfassend die Schritte
- Empfangen mindestens einer ersten MRT-Aufnahme eines Untersuchungsobjekts, wobei die mindestens eine erste MRT-Aufnahme eine Leber oder einen Teil einer Leber des Untersuchungsobjekts ohne eine durch ein Kontrastmittel herbeigeführte Kontrastverstärkung zeigt,
- Empfangen mindestens einer zweiten MRT-Aufnahme eines Untersuchungsobjekts, wobei die mindestens eine zweite MRT-Aufnahme eine Leber oder einen Teil einer Leber des Untersuchungsobjekts zeigt, wobei Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind,
- Zuführen von den empfangenen MRT-Aufnahmen an ein Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts ohne eine durch ein Kontrastmittel herbeigeführte Kontrastverstärkung zeigen und von MRT-Aufnahmen derselben Leber oder demselben Teil der Leber des gleichen Untersuchungsobjekts bei dem Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind, eine oder mehrere MRT-Aufnahmen vorherzusagen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- Erzeugen einer oder mehrerer vorhergesagter MRT-Aufnahmen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

Ein weiterer Gegenstand der vorliegenden Erfindung ist ein System umfassend
- eine Empfangseinheit,
- eine Steuer- und Recheneinheit und
- eine Ausgabeeinheit,
   - wobei die Steuer- und Recheneinheit konfiguriert ist, die Empfangseinheit zu veranlassen, mindestens eine erste MRT-Aufnahme eines Untersuchungsobjekts zu empfangen, wobei die mindestens eine erste MRT-Aufnahme eine Leber oder einen Teil einer Leber des Untersuchungsobjekts ohne eine durch ein Kontrastmittel herbeigeführte Kontrastverstärkung zeigt,
   - wobei die Steuer- und Recheneinheit konfiguriert ist, die Empfangseinheit zu veranlassen, mindestens eine zweite MRT-Aufnahme eines Untersuchungsobjekts zu empfangen, wobei die mindestens eine zweite MRT-Aufnahme dieselbe Leber oder denselben Teil der Leber zeigt, wobei Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind,
   - wobei die Steuer- und Recheneinheit konfiguriert ist, anhand der empfangenen MRT-Aufnahmen eine oder mehrere MRT-Aufnahmen vorherzusagen, wobei die eine oder die mehreren vorhergesagten MRT-Aufnahmen die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
   - wobei die Steuer- und Recheneinheit konfiguriert ist, die Ausgabeeinheit zu veranlassen, eine oder der mehrere vorhergesagte MRT-Aufnahmen anzuzeigen, auszugeben oder in einem Datenspeicher zu speichern.

Ein weiterer Gegenstand der vorliegenden Erfindung ist ein Computerprogrammprodukt umfassend ein Computerprogramm, das in einen Arbeitsspeicher eines Computers geladen werden kann und dort den Computer dazu veranlasst, folgende Schritte ausführen:
- Empfangen mindestens einer ersten MRT-Aufnahme eines Untersuchungsobjekts, wobei die mindestens eine erste MRT-Aufnahme eine Leber oder einen Teil einer Leber des Untersuchungsobjekts ohne eine durch ein Kontrastmittel herbeigeführte Kontrastverstärkung zeigt,
- Empfangen mindestens einer zweiten MRT-Aufnahme desselben Untersuchungsobjekts, wobei die mindestens eine zweite MRT-Aufnahme eine Leber oder einen Teil einer Leber des Untersuchungsobjekts zeigt, wobei Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind,
- Zuführen von den empfangenen MRT-Aufnahmen an ein Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts ohne eine durch ein Kontrastmittel herbeigeführte Kontrastverstärkung zeigen und von MRT-Aufnahmen derselben Leber oder demselben Teil der Leber des gleichen Untersuchungsobjekts bei dem Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind, eine oder mehrere MRT-Aufnahmen vorherzusagen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- Erzeugen einer oder mehrerer vorhergesagter MRT-Aufnahmen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

Ein weiterer Gegenstand der vorliegenden Erfindung ist die Verwendung eines hepatobiliären, paramagnetischen Kontrastmittels in einem MRT-Verfahren, wobei das MRT-Verfahren die folgenden Schritte umfasst:
- Erzeugen mindestens einer ersten MRT-Aufnahme, wobei die mindestens eine erste MRT-Aufnahme die Leber oder einen Teil der Leber ein Untersuchungsobjekts ohne eine durch ein Kontrastmittel herbeigeführte Kontrastverstärkung zeigt,
- Applizieren einer Dosis des hepatobiliären, paramagnetischen Kontrastmittels die höchstens 10% der vollen Dosis des hepatobiliären, paramagnetischen Kontrastmittels entspricht, wobei sich das Kontrastmittel in der Leber des Untersuchungsobjekts verteilt,
- Empfangen mindestens einer zweiten MRT-Aufnahme, wobei die mindestens eine zweite MRT-Aufnahme dieselbe Leber oder denselben Teil der Leber des Untersuchungsobjekts zeigt, wobei Blutgefäße und/oder gesunde Leberzellen in der Leber kontrastverstärkt dargestellt sind,
- Zuführen von den empfangenen MRT-Aufnahmen an ein Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts ohne eine durch ein Kontrastmittel herbeigeführte Kontrastverstärkung zeigen und von MRT-Aufnahmen derselben Leber oder demselben Teil der Leber des gleichen Untersuchungsobjekts bei dem Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind, eine oder mehrere MRT-Aufnahmen vorherzusagen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- Erzeugen einer oder mehrerer vorhergesagter MRT-Aufnahmen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

Ein weiterer Gegenstand ist ein hepatobiliäres, paramagnetisches Kontrastmittel zur Verwendung in einem MRT-Verfahren, wobei das MRT-Verfahren die folgenden Schritte umfasst:
- Erzeugen mindestens einer ersten MRT-Aufnahme, wobei die mindestens eine erste MRT-Aufnahme die Leber oder einen Teil der Leber ein Untersuchungsobjekts ohne eine durch ein Kontrastmittel herbeigeführte Kontrastverstärkung zeigt,
- Applizieren einer Dosis des hepatobiliären, paramagnetischen Kontrastmittels die höchstens 10% der vollen Dosis des hepatobiliären, paramagnetischen Kontrastmittels entspricht, wobei sich das Kontrastmittel in der Leber des Untersuchungsobjekts verteilt,
- Empfangen mindestens einer zweiten MRT-Aufnahme, wobei die mindestens eine zweite MRT-Aufnahme dieselbe Leber oder denselben Teil der Leber des Untersuchungsobjekts zeigt, wobei Blutgefäße und/oder gesunde Leberzellen in der Leber kontrastverstärkt dargestellt sind,
- Zuführen von den empfangenen MRT-Aufnahmen an ein Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts ohne eine durch ein Kontrastmittel herbeigeführte Kontrastverstärkung zeigen und von MRT-Aufnahmen derselben Leber oder demselben Teil der Leber des gleichen Untersuchungsobjekts bei dem Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind, eine oder mehrere MRT-Aufnahmen vorherzusagen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- Erzeugen einer oder mehrerer vorhergesagter MRT-Aufnahmen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

Ein weiterer Gegenstand ist ein Kit umfassend ein Kontrastmittel und ein erfindungsgemäßes Computerprogrammprodukt.

Die Erfindung wird nachstehend näher erläutert, ohne zwischen den Erfindungsgegenständen (Verfahren, System, Computerprogrammprodukt, Verwendung, Kontrastmittel zur Verwendung, Kit) zu unterscheiden. Die nachfolgenden Erläuterungen sollen vielmehr für alle Erfindungsgegenstände in analoger Weise gelten, unabhängig davon, in welchem Kontext (Verfahren, System, Computerprogrammprodukt, Verwendung, Kontrastmittel zur Verwendung, Kit) sie erfolgen.

Wenn in der vorliegenden Beschreibung oder in den Patentansprüchen Schritte in einer Reihenfolge genannt sind, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar.

Die vorliegende Erfindung erzeugt eine bzw. mehrere künstliche MRT-Aufnahme(n) einer Leber oder eines Teils einer Leber eines Untersuchungsobjekts, die eine bzw. mehrere MRT-Aufnahme(n) durch ein Kontrastmittel herbeigeführte Kontrastverstärkung zeigen. Die Kontrastverstärkung entspricht derjenigen, die erhalten wird, wenn 100% der vollen, empfohlenen Dosis appliziert wird. Die künstlichen MRT-Aufnahme(n) werden auf der Basis von MRT-Aufnahmen erstellt, die nicht bzw. nur schwach kontrastverstärkt sind (d.h. es wurde nur höchstens 10% der vollen, empfohlenen Dosis appliziert). D.h. die künstliche(n) MRT-Aufnahme(n) können mit Hilfe eines selbstlernenden Algorithmus erstellt werden und imitieren MRT-Aufnahme(n) von der Leber oder eines Teiles der Leber des Untersuchungsobjekts mit einer durch ein Kontrastmittel herbeigeführten Kontrastverstärkung, die durch Applikation einer Dosis die 100% der vollen Dosis des Kontrastmittels entspricht, herbeigeführt werden kann. Insgesamt kann durch die Erfindung die Dosis des Kontrastmittels zum Wohle der Patientenverträglichkeit deutlich reduziert werden, ohne dass dies die Ergebnisfindung beeinträchtigt.

Das "Untersuchungsobjekt" ist üblicherweise ein Lebewesen, vorzugsweise ein Säugetier, ganz besonders bevorzugt ein Mensch.

Ein Teil des Untersuchungsobjekts wird einer kontrastverstärkten Magnetresonanztomographie-Untersuchung unterzogen. Der "Untersuchungsbereich", auch Aufnahmevolumen (engl.: *field of view,* FOV) genannt, stellt insbesondere ein Volumen dar, welches in den Magnetresonanzaufnahmen abgebildet wird. Der Untersuchungsbereich wird typischerweise durch einen Radiologen, beispielsweise auf einer Übersichtsaufnahme (engl.: *localizer*) festgelegt. Selbstverständlich kann der Untersuchungsbereich alternativ oder zusätzlich auch automatisch, beispielsweise auf Grundlage eines ausgewählten Protokolls, festgelegt werden. Der Untersuchungsbereich umfasst zumindest einen Teil der Leber des Untersuchungsobjekts.

Der Untersuchungsbereich wird in ein Grundmagnetfeld eingebracht

Dem Untersuchungsobjekt wird ein hepatobiliäres, paramagnetisches Kontrastmittel verabreicht, das sich in dem Untersuchungsbereich verteilt. Das Kontrastmittel wird vorzugsweise intravenös als Bolus, gewichtsadaptiert dosiert verabreicht.

Unter einem hepatobiliären, paramagnetischen "Kontrastmittel" wird ein Stoff oder Stoffgemisch verstanden, dessen Anwesenheit in einer Magnetresonanzmessung zu einem veränderten Signal führt. Vorzugsweise führt das Kontrastmittel zu einer Verkürzung der T1-Relaxationszeit.

Vorzugsweise ist das Kontrastmittel ein hepatobiliäre, paramagnetische Kontrastmittel wie beispielsweise Gd-EOB-DTPA oder Gd-BOPTA.

In einer besonders bevorzugten Ausführungsform handelt es sich bei dem Kontrastmittel um einen Stoff oder ein Stoffgemisch mit der Gadoxetsäure oder einem Salz der Gadoxetsäure als kontrastverstärkenden Wirkstoff. Ganz besonders bevorzugt handelt es sich um das Dinatriumsalz der Gadoxetsäure (Gd-EOB-DTPA Dinatrium).

Im Sinne der Erfindung bezeichnet "eine Dosis die 100% der vollen Dosis des Kontrastmittels entspricht" beispielsweise im Zusammenhang mit Gd-EOB-DTPA Dinatrium, dass 0,025 mmol/kg Körpergewicht bzw. 0,1 ml/kg Körpergewicht appliziert wird. Entsprechend bezeichnet "höchstens 10% der vollen Dosis", dass höchstens 0,0025 mmol/kg Körpergewicht bzw. höchstens 0,01 ml/kg Körpergewicht appliziert wird. MRT-Aufnahmen die mit der vollen Dosis generiert werden, werden nachfolgend auch MRT-Aufnahmen mit starker Kontrastverstärkung bezeichnet. MRT-Aufnahmen, die mit "höchstens 10% der vollen Dosis" generiert werden, werden nachfolgend auch MRT-Aufnahmen mit schwacher Kontrastverstärkung bezeichnet.

Der Untersuchungsbereich wird einem MRT-Verfahren unterzogen und dabei werden MRT-Aufnahmen erzeugt (gemessen), die den Untersuchungsbereich während der Untersuchungsphase zeigen.

Die gemessenen MRT-Aufnahmen können als zweidimensionale Bildaufnahmen vorliegen, die eine Schnittebene durch das Untersuchungsobjekt zeigen. Die gemessenen MRT-Aufnahmen können als Stapel zweidimensionaler Bildaufnahme vorliegen, wobei jede einzelne Bildaufnahme des Stapels eine andere Schnittebene zeigt. Die gemessenen MRT-Aufnahmen können als dreidimensionale Aufnahmen (3D-Aufnahmen) vorliegen. Aus Gründen der einfacheren Darstellung wird die Erfindung an einigen Stellen der vorliegenden Beschreibung anhand des Vorliegens von zweidimensionalen MRT-Aufnahmen erläutert, ohne die Erfindung jedoch auf zweidimensionale MRT-Aufnahmen beschränken zu wollen. Dem Fachmann ist klar, wie sich das jeweils Beschriebene auf Stapel zweidimensionaler Bildaufnahmen und auf 3D-Aufnahmen übertragen lässt (siehe hierzu z.B. M. Reisler, W. Semmler: Magnetresonanztomographie, Springer Verlag, 3. Auflage, 2002, ISBN: 978-3-642-63076-7).

Nach der intravenösen Applikation eines hepatobiliären, paramagnetischen Kontrastmittels in Form eines Bolus erreicht das Kontrastmittel die Leber zunächst über die Arterien. Diese sind in den entsprechenden MRT-Aufnahmen kontrastverstärkt dargestellt. Die Phase, in der die Leberarterien in MRT-Aufnahmen kontrastverstärkt dargestellt sind, wird hier als "arterielle Phase" bezeichnet. Diese Phase beginnt unmittelbar nach der Applikation des Kontrastmittels und dauert üblicherweise 15 bis 25 Sekunden.

Anschließend erreicht das Kontrastmittel die Leber über die Lebervenen. Während der Kontrast in den Leberarterien bereits abnimmt, erreicht der Kontrast in den Lebervenen ein Maximum. Die Phase, in der die Lebervenen in MRT-Aufnahmen kontrastverstärkt dargestellt sind, wird hier als "venöse Phase" bezeichnet. Diese Phase kann bereits während der arteriellen Phase beginnen und sich mit dieser überlagern. Üblicherweise startet diese Phase 20 bis 30 Sekunden nach der intravenösen Applikation und dauert üblicherweise 40 bis 60 Sekunden.

An die venöse Phase schließt sich die "Spätphase" an, in der der Kontrast in den Leberarterien weiter absinkt, der Kontrast in den Lebervenen ebenfalls absinkt und der Kontrast in den gesunden Leberzellen allmählich anstiegt. Diese Phase beginnt üblicherweise 70 bis 90 Sekunden nach der Applikation des Kontrastmittels und dauert üblicherweise 100 bis 120 Sekunden.

Die arterielle Phase, die venöse Phase und die Spätphase werden zusammenfassend auch als "dynamische Phase" bezeichnet.

10-20 Minuten nach seiner Injektion führt ein hepatobiliäres Kontrastmittel zu einer deutlichen Signalverstärkung im gesunden Leberparenchym. Diese Phase wird hier als "hepatobiliäre Phase" bezeichnet. Das Kontrastmittel wird aus den Leberzellen nur langsam ausgeschieden; dementsprechend kann die hepatobiliäre Phase zwei Stunden und mehr andauern.

Die genannten Phasen sind beispielsweise in den folgenden Publikationen näher beschrieben: J. Magn. Reson. Imaging, 2012, 35(3): 492-511, doi:10.1002/jmri.22833; Clujul Medical, 2015, Vol. 88 no. 4: 438-448, DOI: 10.15386/cjmed-414; Journal of Hepatology, 2019, Vol. 71: 534-542, http://dx.doi.org/10.1016/j.jhep.2019.05.005).

Als "erste MRT-Aufnahme" wird in dieser Beschreibung eine MRT-Aufnahme bezeichnet, bei der eine Leber oder einen Teil einer Leber des Untersuchungsobjekts ohne eine durch ein Kontrastmittel herbeigeführte Kontrastverstärkung dargestellt ist. D.h. die MRT-Aufnahme erfolgt ohne vorherige Applikation eines Kontrastmittels.

Als "zweite MRT-Aufnahme" wird in dieser Beschreibung eine MRT-Aufnahme bezeichnet, die die Leber oder einen Teil einer Leber des Untersuchungsobjekts zeigt, wobei Blutgefäße und/oder gesunde Leberzellen in der Leber, nach Applikation einer Dosis die höchstens 10%, vorzugsweise höchstens 8% und noch bevorzugter höchstens 5% der vollen Dosis eines hepatobiliären, paramagnetischen Kontrastmittels entspricht, kontrastverstärkt dargestellt sind. D.h. es können Aufnahmen von der dynamischen Phase und/oder der hepatobiliären Phase aufgenommen werden.

Vorzugsweise handelt es sich bei der mindestens einen "zweiten" MRT-Aufnahme um mindestens eine MRT-Aufnahme, die während der dynamischen Phase gemessen worden ist. Besonders bevorzugt handelt es sich um jeweils mindestens eine MRT-Aufnahme, die während der arteriellen Phase, der venösen Phase und/oder während der Spätphase gemessen worden ist. Ganz besonders bevorzugt handelt es sich um jeweils mindestens eine MRT-Aufnahme, die während der arteriellen Phase gemessen worden ist. Vorzugsweise handelt es sich bei der mindestens einen ersten MRT-Aufnahme um eine T1-gewichtete Darstellung.

Bei Verwendung eines paramagnetischen Kontrastmittels sind die Blutgefäße in der mindestens einen ersten MRT-Aufnahme aufgrund der Kontrastverstärkung durch eine hohe Signalintensität gekennzeichnet (signalreiche Darstellung). Diejenigen (zusammenhängenden) Strukturen innerhalb einer "zweiten" MRT-Aufnahme, die eine Signalintensität innerhalb einer empirisch ermittelbaren Bandbreite aufweisen, lassen sich damit Blutgefäßen zuordnen. Damit liegt mit der mindestens einen "zweiten" MRT-Aufnahme eine Information darüber vor, wo in den MRT-Aufnahmen Blutgefäße dargestellt sind bzw. welche Strukturen in den MRT-Aufnahmen auf Blutgefäße (Arterien und/oder Venen) zurückzuführen sind.

Die "erste MRT-Aufnahme" und die "zweite MRT-Aufnahme" werden einem Vorhersagemodell zugeführt. Das Vorhersagemodell ist ein Modell, das konfiguriert ist, auf Basis der empfangenden MRT-Aufnahmen, eine oder mehrere MRT-Aufnahmen vorherzusagen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann.

Dabei bedeutet der Begriff "Vorhersage", dass eine bzw. mehrere künstliche MRT-Aufnahme(n), die die Leber oder einen Teil davon von einem Untersuchungsobjekt mit starker Kontrastverstärkung zeigen, unter Verwendung der MRT-Aufnahmen, die den gleichen Untersuchungsbereich mit durch ein Kontrastmittel herbeigeführte schwachen Kontrastverstärkung bzw. mit gar keiner Kontrastverstärkung zeigen, berechnet wird.

Das Vorhersagemodell wurde vorzugsweise mit Hilfe eines selbstlernenden Algorithmus in einem überwachten maschinellen Lernen erstellt. Zum Lernen werden Trainingsdaten verwendet, die eine Vielzahl an MRT-Aufnahmen der dynamischen Phase und/oder der hepatobiliären Phase (vorzugsweise der dynamischen Phase und noch bevorzugter der arteriellen Phase) der Leber oder eines Teils der Leber von einem Untersuchungsobjekt umfassen. Diese MRT-Aufnahmen wurden generiert, indem dem Untersuchungsobjekt höchstens 10%, bevorzugt höchstens 8% und besonders bevorzugt höchstens 5% der vollen Dosis eines hepatobiliären, paramagnetischen Kontrastmittels verabreicht wurde. Weitere Trainingsdaten von den gleichen Untersuchungsobjekten wurden verwendet, bei denen die MRT-Aufnahmen nach vorheriger Applikation von 100% der vollen Dosis des gleichen Kontrastmittels generiert wurden. Somit lagen Trainingsdaten von gleichen Untersuchungsobjekt vor mit unterschiedlichen schwachen bzw. starken Kontrastverstärkungen.

Weiter wurde zusätzlich auch ein Trainingsdaten verwendet, die von MRT-Aufnahmen der gleichen Leber bzw. Teils einer Leber des gleichen Untersuchungsobjekts erstellt wurden und bei denen keine Kontrastverstärkung vorlag, d.h. die ohne Applikation eines Kontrastmittels generiert wurden.

Der selbstlernende Algorithmus erzeugt beim maschinellen Lernen ein statistisches Modell, das auf den Trainingsdaten beruht. Das heißt, es werden nicht einfach die Beispiele auswendig gelernt, sondern der Algorithmus "erkennt" Muster und Gesetzmäßigkeiten in den Trainingsdaten. So kann der Algorithmus auch unbekannte Daten beurteilen. Validierungsdaten können verwendet werden, um die Güte der Beurteilung unbekannter Daten zu prüfen.

Das Trainieren des selbstlernenden Algorithmus erfolgt mittels überwachten Lernens (engl.: *supervised learning*), d.h. dem Algorithmus werden nacheinander MRT-Aufnahmen mit keiner bzw. schwacher Kontrastverstärkung der dynamischen Phase und/oder der hepatobiliären Phase präsentiert und es wird ihm mitgeteilt, welche MRT-Aufnahmen mit stark kontrastverstärkten MRT-Aufnahmen damit verbunden sind. Der Algorithmus lernt dann eine Beziehung zwischen den MRT-Aufnahmen mit keiner bzw. schwacher Kontrastverstärkung und den MRT-Aufnahmen mit starker Kontrastverstärkung, um für MRT-Aufnahmen mit keiner bzw. schwacher Kontrastverstärkung ein oder mehrere MRT-Aufnahmen mit starker Kontrastverstärkung vorherzusagen.

Selbstlernende Algorithmen, die mittels überwachten Lernens trainiert werden, sind vielfältig im Stand der Technik beschrieben (siehe z.B. C. Perez: Machine Learning Techniques: Supervised Learning and Classification, Amazon Digital Services LLC - Kdp Print Us, 2019, ISBN 1096996545, 9781096996545).

Vorzugsweise handelt es sich bei dem Vorhersagemodell um ein künstliches neuronales Netz.

Ein solches künstliches neuronales Netzwerk umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen dienen zum Empfangen von digitalen MRT-Aufnahmen als Eingangswerte. Normalerweise gibt es ein Eingangsneuron für jedes Pixel oder Voxel einer digitalen MRT-Aufnahme. Es können zusätzliche Eingangsneuronen für zusätzliche Eingangswerte (z.B. Informationen zum Untersuchungsbereich, zum Untersuchungsobjekt und/oder zu Bedingungen, die bei der Erzeugung der MRT-Aufnahmen herrschten) vorhanden sein.

In einem solchen Netzwerk dienen die Ausgangsneuronen dazu, für eine erste und eine zweite MRT-Aufnahme eine dritte künstliche MRT-Aufnahme zu generieren. Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN).

Ein Convolutional Neural Network ist in der Lage, Eingabedaten in Form einer Matrix zu verarbeiten. Dies ermöglicht es, als Matrix dargestellte digitale MRT-Aufnahmen (z.B. Breite x Höhe x Farbkanäle) als Eingabedaten zu verwenden. Ein normales neuronales Netz z.B. in Form eines Multi-Layer-Perceptrons (MLP) benötigt dagegen einen Vektor als Eingabe, d.h. um eine MRT-Aufnahme als Eingabe zu verwenden, müssten die Pixel oder Voxel der MRT-Aufnahme in einer langen Kette hintereinander ausgerollt werden. Dadurch sind normale neuronale Netze z.B. nicht in der Lage, Objekte in einer MRT-Aufnahme unabhängig von der Position des Objekts in der MRT-Aufnahme zu erkennen. Das gleiche Objekt an einer anderen Position in der MRT-Aufnahme hätte einen völlig anderen Eingabevektor.

Ein CNN besteht im Wesentlichen aus Filtern (Convolutional Layer) und Aggregations-Schichten (Pooling Layer), die sich abwechselnd wiederholen, und am Ende aus einer Schicht oder mehreren Schichten von "normalen" vollständig verbundenen Neuronen (Dense / Fully Connected Layer).

Bei der Analyse von Sequenzen (Abfolgen von MRT-Aufnahme) können Raum und Zeit als äquivalente Dimensionen behandelt und z.B. über 3D-Faltungen verarbeitet werden. Dies wurde in den Arbeiten von Baccouche et al. (Sequential Deep Learning for Human Action Recognition; International Workshop on Human Behavior Understanding, Springer 2011, pages 29-39) und Ji et al. (3D Convolutional Neural Networks for Human Action Recognition, IEEE Transactions on Pattern Analysis and Machine Intelligence, 35(1), 221-231) gezeigt. Ferner kann man verschiedene Netzwerke trainieren, die für Zeit und Raum verantwortlich sind, und schließlich die Merkmale verschmelzen, wie in Veröffentlichungen von Karpathy et al. (Large-scale Video Classification with Convolutional Neural Networks; Proceedings of the IEEE conference on Computer Vision and Pattern Recognition, 2014, pages 1725-1732) und Simonyan & Zisserman (Two-stream Convolutional Networks for Action Recognition in Videos; Advances in Neural Information Processing Systems, 2014, pages 568-576) beschrieben ist.

Rekurrente neuronale Netze (RNNs) sind eine Familie von so genannten Feedforward Neuronalen Netzen, die Rückkopplungsverbindungen zwischen Schichten enthalten. RNNs ermöglichen die Modellierung sequentieller Daten durch gemeinsame Nutzung von Parameterdaten über verschiedene Teile des neuronalen Netzwerks. Die Architektur für ein RNN enthält Zyklen. Die Zyklen repräsentieren den Einfluss eines gegenwärtigen Werts einer Variablen auf ihren eigenen Wert zu einem zukünftigen Zeitpunkt, da mindestens ein Teil der Ausgabedaten von der RNN als Rückkopplung zur Verarbeitung nachfolgender Eingaben in einer Sequenz verwendet wird.

Details sind dem Stand der Technik zu entnehmen (siehe z.B.: S. Khan et al.: A Guide to Convolutional Neural Networks for Computer Vision, Morgan & Claypool Publishers 2018, ISBN 1681730227, 9781681730226).

Das Trainieren des neuronalen Netzes kann beispielsweise mittels eines Backpropagation-Verfahrens durchgeführt werden. Dabei wird für das Netz eine möglichst zuverlässige Abbildung von gegebenen Eingabevektoren auf gegebene Ausgabevektoren angestrebt. Die Qualität der Abbildung wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich der Beziehung der schwach und der stark kontrastverstärkte MRT-Aufnahmen der dynamischen und/oder hepatobiliären Phase und MRT-Aufnahmen ohne Kontrastverstärkung, die verwendet werden können, um eine oder mehrere MRT-Aufnahmen vorherzusagen, die einen Untersuchungsbereich mit starker Kontrastverstärkung zeigen und die nur mittels MRT-Aufnahmen die keine bzw. eine schwache Kontrastverstärkung aufweisen, berechnet werden.

Eine Kreuzvalidierungsmethode kann verwendet werden, um die Daten in Trainings- und Validierungsdatensätze aufzuteilen. Der Trainingsdatensatz wird beim Backpropagations-Training der Netzwerkgewichte verwendet. Der Validierungsdatensatz wird verwendet, um zu überprüfen, mit welcher Vorhersagegenauigkeit sich das trainierte Netzwerk auf unbekannte Mehrzahlen von MRT-Aufnahmen anwenden lässt.

Wie bereits angedeutet, können zum Trainieren, Validieren und Vorhersagen auch weitere Informationen zum Untersuchungsobjekt, zum Untersuchungsbereich und/oder zu Untersuchungsbedingungen verwendet werden.

Beispiele für Informationen zum Untersuchungsobjekt sind: Geschlecht, Alter, Gewicht, Größe, Anamnese, Art und Dauer und Menge bereits eingenommener Medikamente, Blutdruck, zentralvenöser Druck, Atemfrequenz, Serum, Albumin, Total Bilirubin, Blutzucker, Eisengehalt, Atemkapazität und dergleichen. Diese können z.B. auch einer Datenbank bzw. einer elektronischen Patientenakte entnommen werden.

Beispiele für Informationen zum Untersuchungsbereich sind: Vorerkrankungen, Operationen, Teilresektion, Lebertransplantation, Eisenleber, Fettleber und dergleichen.

Es ist denkbar, dass die empfangenen MRT-Aufnahmen, einer Bewegungskorrektur unterzogen werden, bevor sie dem Vorhersagemodell zugeführt werden. Eine solche Bewegungskorrektur sorgt dafür, dass ein Pixel oder Voxel einer ersten MRT-Aufnahme denselben Untersuchungsbereich zeigt, wie das entsprechende Pixel oder Voxel einer zweiten, zeitlich nachgelagerten MRT-Aufnahme. Bewegungskorrekturverfahren sind im Stand der Technik beschrieben (siehe zum Beispiel: EP3118644, EP3322997, US20080317315, US20170269182, US20140062481, EP2626718).

Ein Gegenstand der vorliegenden Erfindung ist ein System, mit dem das erfindungsgemäße Verfahren ausgeführt werden kann.

Das System umfasst eine Empfangseinheit, eine Steuer- und Recheneinheit und eine Ausgabeeinheit.

Es ist denkbar, dass die genannten Einheiten Bestandteile eines einzigen Computersystems sind; es ist aber auch denkbar, dass die genannten Einheiten Bestandteile von mehreren separaten Computersystemen sind, die über ein Netzwerk miteinander verbunden sind, um Daten und/oder Steuersignale von einer Einheit zu einer anderen Einheit zu übermitteln.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Computersysteme von heute werden häufig in Desktop-PCs, Portable PCs, Laptops, Notebooks, Netbooks und Tablet-PCs und so genannte Handhelds (z.B. Smartphone) unterteilt; alle diese Systeme können zur Ausführung der Erfindung genutzt werden.

Eingaben in das Computersystem erfolgen über Eingabemittel wie beispielsweise eine Tastatur, eine Maus, ein Mikrofon, ein berührungsempfindliches Display und/oder dergleichen.

Das erfindungsgemäße System ist konfiguriert, mindestens eine erste MRT-Aufnahme von der Leber oder von einem Teil der Leber eines Untersuchungsobjekts ohne Kontrastverstärkung und mindestens eine zweite MRT-Aufnahme derselben Leber bzw. einem Teil davon des gleichen Untersuchungsobjekts mit schwacher Kontrastverstärkung der dynamischen und/oder hepatobiliiären Phase zu empfangen und auf Basis dieser Daten und ggf. weiterer Daten eine oder mehrere MRT-Aufnahmen zu erzeugen (vorherzusagen, zu berechnen), die den Untersuchungsbereich d.h. die Leber oder Teile davon mit starker Kontrastverstärkung zeigen.

Die Steuer- und Recheneinheit dient der Steuerung der Empfangseinheit, der Koordinierung der Daten- und Signalflüsse zwischen verschiedenen Einheiten und der Prozessierung und Erzeugung von MRT-Aufnahmen. Es ist denkbar, dass mehrere Steuer- und Recheneinheiten vorhanden sind.

Die Empfangseinheit dient zum Empfang von MRT-Aufnahmen. Die MRT-Aufnahmen können beispielsweise von einer Magnetresonanzanlage übermittelt werden oder aus einem Datenspeicher ausgelesen werden. Die Magnetresonanzanlage kann ein Bestandteil des erfindungsgemäßen Systems sein. Denkbar ist aber auch, dass das erfindungsgemäße System ein Bestandteil einer Magnetresonanzanlage ist.

Von der Empfangseinheit werden die mindestens eine erste MRT-Aufnahme und die mindestens eine zweite MRT-Aufnahme und ggf. weitere Daten an die Steuer- und Recheneinheit übermittelt.

Die Steuer- und Recheneinheit ist konfiguriert, anhand der MRT-Aufnahmen, die einen Untersuchungsbereich mit keiner bzw. schwacher Kontrastverstärkung der dynamischen und der hepatobiliären Phase zeigen, eine oder mehrere MRT-Aufnahmen vorherzusagen, wobei die vorhergesagten MRT-Aufnahmen den Untersuchungsbereich mit starker Kontrastverstärkung zeigen. Vorzugsweise kann in einen Arbeitsspeicher der Steuer- und Recheneinheit ein Vorhersagemodell geladen werden, mit dem die MRT-Aufnahmen mit starker Kontrastverstärkung berechnet werden. Das Vorhersagemodell wurde vorzugsweise mit Hilfe eines selbstlernenden Algorithmus mittels überwachtem Lernen erzeugt (trainiert).

Über die Ausgabeeinheit kann die mindestens eine vorhergesagte MRT-Aufnahme angezeigt werden (zum Beispiel auf einem Monitor), ausgegeben werden (z.B. über einen Drucker) oder in einem Datenspeicher gespeichert werden.

Die Erfindung wird nachstehend anhand von Figuren näher erläutert, ohne die Erfindung auf die in den Figuren gezeigten Merkmale oder Merkmalskombinationen beschränken zu wollen.

Es zeigen:

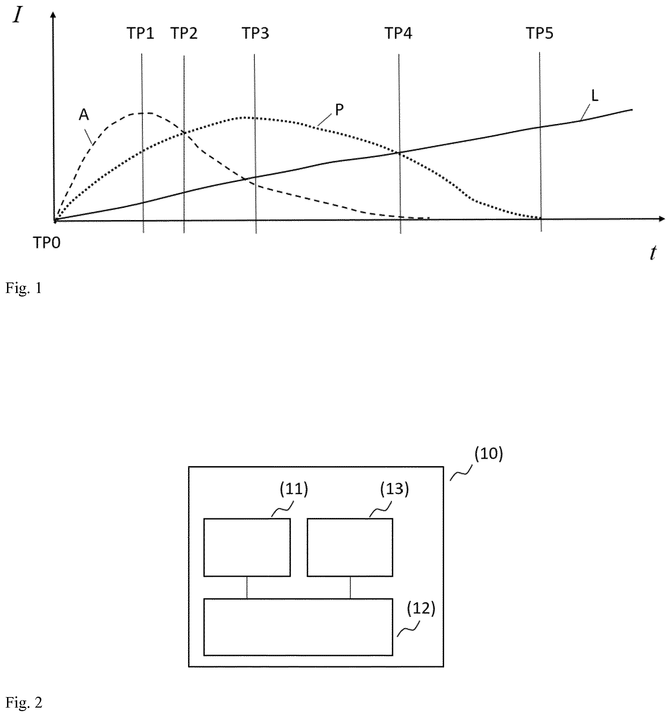

Figur 1 zeigt schematisch den zeitlichen Verlauf der Konzentrationen von Kontrastmittel in den Leberarterien (A), den Lebervenen (P) und den Leberzellen (L). Die Konzentrationen sind in Form der Signalintensitäten I in den genannten Arealen (Leberarterien, Lebervenen, Leberzellen) bei der Magnetresonanzmessung als Funktion der Zeit t dargestellt. Bei einer intravenösen Bolusinjektion steigt die Konzentration des Kontrastmittels in den Leberarterien (A) als erstes an (gestrichelte Kurve). Die Konzentration durchläuft ein Maximum und sinkt dann ab. Die Konzentration in den Lebervenen (P) steigt langsamer an als in den Leberarterien und erreicht ihr Maximum später (gepunktete Kurve). Die Konzentration des Kontrastmittels in den Leberzellen steigt langsam an (durchgezogene Kurve) und erreicht ihr Maximum erst zu einem sehr viel späteren Zeitpunkt (in der Figur 1 nicht dargestellt). Es lassen sich einige charakteristische Zeitpunkte definieren: Zum Zeitpunkt TP0 wird Kontrastmittel intravenös als Bolus appliziert. Zum Zeitpunkt TP1 erreicht die Konzentration (die Signalintensität) des Kontrastmittels in den Leberarterien ihr Maximum. Zum Zeitpunkt TP2 schneiden sich die Kurven der Signalintensitäten bei den Leberarterien und den Lebervenen. Zum Zeitpunkt TP3 durchläuft die Konzentration (die Signalintensität) des Kontrastmittels in den Lebervenen ihr Maximum. Zum Zeitpunkt TP4 schneiden sich die Kurven der Signalintensitäten bei den Leberarterien und den Leberzellen. Zum Zeitpunkt T5 sind die Konzentrationen in den Leberarterien und den Lebervenen auf ein Niveau gesunken, bei dem sie keine messbare Kontrastverstärkung mehr verursachen.
Figur 2 zeigt schematisch eine bevorzugte Ausführungsform des erfindungsgemäßen Systems. Das System (10) umfasst eine Empfangseinheit (11), eine Steuer- und Recheneinheit (12) und eine Ausgabeeinheit (13).

Die Steuer- und Recheneinheit (12) ist konfiguriert, die Empfangseinheit (11) zu veranlassen, mindestens eine erste MRT-Aufnahme eines Untersuchungsobjekts zu empfangen, wobei die mindestens eine erste MRT-Aufnahme eine Leber oder einen Teil einer Leber des Untersuchungsobjekts ohne eine durch ein Kontrastmittel herbeigeführte Kontrastverstärkung zeigt,
Die Steuer- und Recheneinheit (12) ist ferner konfiguriert, die Empfangseinheit (11) zu veranlassen, mindestens eine zweite MRT-Aufnahme eines Untersuchungsobjekts zu empfangen, wobei die mindestens eine zweite MRT-Aufnahme dieselbe Leber oder denselben Teil der Leber zeigt, wobei Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind,
Die Steuer- und Recheneinheit (12) ist ferner konfiguriert, anhand der empfangenen MRT-Aufnahmen eine oder mehrere MRT-Aufnahmen vorherzusagen, wobei die eine oder die mehreren vorhergesagten MRT-Aufnahmen die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
Die Steuer- und Recheneinheit (12) ist ferner konfiguriert, die Ausgabeeinheit (13) zu veranlassen, die mindestens eine vorhergesagte MRT-Aufnahme anzuzeigen, auszugeben oder in einem Datenspeicher zu speichern.

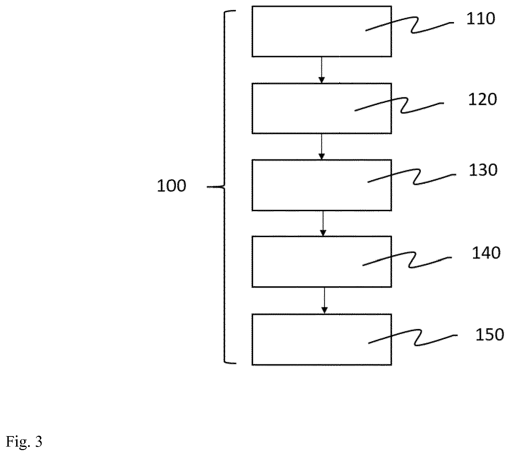

Figur 3 zeigt schematisch und beispielhaft eine Ausführungsform des erfindungsgemäßen Verfahrens. Das Verfahren (100) umfasst die Schritte:
- (110) Empfangen mindestens einer ersten MRT-Aufnahme eines Untersuchungsobjekts, wobei die mindestens eine erste MRT-Aufnahme eine Leber oder einen Teil einer Leber des Untersuchungsobjekts ohne eine durch ein Kontrastmittel herbeigeführte Kontrastverstärkung zeigt,
- (120) Empfangen mindestens einer zweiten MRT-Aufnahme eines Untersuchungsobjekts, wobei die mindestens eine zweite MRT-Aufnahme eine Leber oder einen Teil einer Leber des Untersuchungsobjekts zeigt, wobei Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind,
- (130) Zuführen von den empfangenen MRT-Aufnahmen an ein Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts ohne eine durch ein Kontrastmittel herbeigeführte Kontrastverstärkung zeigen und von MRT-Aufnahmen derselben Leber oder demselben Teil der Leber des gleichen Untersuchungsobjekts bei dem Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind, eine oder mehrere MRT-Aufnahmen vorherzusagen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- (140) Erzeugen einer oder mehrerer vorhergesagter MRT-Aufnahmen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- (150) Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

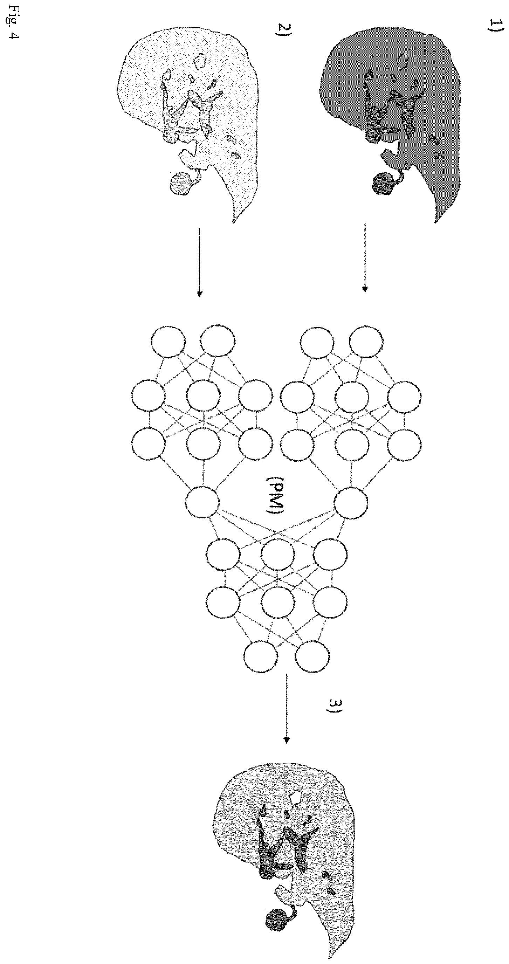

Figur 4 zeigt beispielhaft und schematisch eine weitere Ausführungsform der vorliegenden Erfindung. Es wird eine erste MRT-Aufnahme (1) bereitgestellt, wobei die erste MRT-Aufnahme eine Leber oder einen Teil einer Leber eines Untersuchungsobjekts ohne eine durch ein Kontrastmittel herbeigeführte Kontrastverstärkung zeigt.,

Es wird eine zweite MRT-Aufnahme (2) bereitgestellt, wobei die zweite MRT-Aufnahme dieselbe Leber oder denselben Teil der Leber zeigt wie die erste MRT-Aufnahme, wobei die Blutgefäße (arterielle Phase) durch ein Kontrastmittel schwach kontrastverstärkt (signalverstärkt) dargestellt ist. Zur Generierung der MRT-Aufnahmen wurde dem Untersuchungsobjekt eine Dosis die höchstens 10% der vollen Dosis des Kontrastmittels entspricht, appliziert.

Die erste MRT-Aufnahme (1) und die zweite MRT-Aufnahme (2) werden einem Vorhersagemodell (PM) zugeführt.

Das Vorhersagemodell (PM) ist konfiguriert, auf Basis der ersten MRT-Aufnahme (1) und der zweiten MRT-Aufnahme (2) eine dritte MRT-Aufnahme (3) zu erzeugen, die eine MRT-Aufnahme mit einer starken Kontrastverstärkung zeigt,

Das Vorhersagemodell wurde vorzugsweise mit Hilfe eines selbstlernenden Algorithmus in einem überwachten maschinellen Lernen mit einem Trainingsdatensatz erstellt. Der Trainingsdatensatz umfasst eine Vielzahl an ersten MRT-Aufnahmen, zweiten MRT-Aufnahmen und den dazugehörigen dritten MRT-Aufnahme, wobei die dritte MRT-Aufnahme tatsächlich aufgenommen wurden. D.h. für die gleichen Untersuchungsobjekte wurden MRT-Aufnahmen generiert, die durch Applikation einer Dosis die 100% der vollen Dosis des Kontrastmittels entspricht, kontrastverstärkt wurden.

Der selbstlernende Algorithmus erzeugt beim maschinellen Lernen ein statistisches Modell, das auf den Trainingsdaten beruht. Das heißt, es werden nicht einfach die Beispiele auswendig gelernt, sondern der Algorithmus "erkennt" Muster und Gesetzmäßigkeiten in den Trainingsdaten. So kann der Algorithmus auch unbekannte Daten beurteilen. Validierungsdaten können verwendet werden, um die Güte der Beurteilung unbekannter Daten zu prüfen.

Das Trainieren des selbstlernenden Algorithmus erfolgt mittels überwachten Lernens (engl.: supervised learning), d.h. dem Algorithmus werden erste und zweite MRT-Aufnahmen präsentiert und es wird ihm mitgeteilt, welche dritten MRT-Aufnahmen mit den jeweiligen ersten und zweiten MRT-Aufnahmen verbunden sind. Der Algorithmus lernt dann eine Beziehung zwischen den MRT-Aufnahmen, um für unbekannte erste und zweite MRT-Aufnahmen dritte MRT-Aufnahmen vorherzusagen (zu berechnen).

Selbstlernende Algorithmen, die mittels überwachten Lernens trainiert werden, sind vielfältig im Stand der Technik beschrieben (siehe z.B. C. Perez: Machine Learning Techniques: Supervised Learning and Classification, Amazon Digital Services LLC - Kdp Print Us, 2019, ISBN 1096996545, 9781096996545).

Vorzugsweise handelt es sich bei dem Vorhersagemodell um ein künstliches neuronales Netz.

Ein solches künstliches neuronales Netzwerk umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen dienen zum Empfangen von digitalen MRT-Aufnahmen als Eingangswerte. Normalerweise gibt es ein Eingangsneuron für jedes Pixel oder Voxel einer digitalen MRT-Aufnahme. Es können zusätzliche Eingangsneuronen für zusätzliche Eingangswerte (z.B. Informationen zum Untersuchungsbereich, zum Untersuchungsobjekt und/oder zu Bedingungen, die bei der Erzeugung der MRT-Aufnahmen herrschten) vorhanden sein.

In einem solchen Netzwerk dienen die Ausgangsneuronen dazu, für eine erste und eine zweite MRT-Aufnahme eine dritte MRT-Aufnahme zu generieren.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN).

Ein Convolutional Neural Network ist in der Lage, Eingabedaten in Form einer Matrix zu verarbeiten. Dies ermöglicht es, als Matrix dargestellte digitale MRT-Aufnahmen (z.B. Breite x Höhe x Farbkanäle) als Eingabedaten zu verwenden. Ein normales neuronales Netz z.B. in Form eines Multi-Layer-Perceptrons (MLP) benötigt dagegen einen Vektor als Eingabe, d.h. um eine MRT-Aufnahme als Eingabe zu verwenden, müssten die Pixel oder Voxel der MRT-Aufnahme in einer langen Kette hintereinander ausgerollt werden. Dadurch sind normale neuronale Netze z.B. nicht in der Lage, Objekte in einer MRT-Aufnahme unabhängig von der Position des Objekts in der MRT-Aufnahme zu erkennen. Das gleiche Objekt an einer anderen Position in der MRT-Aufnahme hätte einen völlig anderen Eingabevektor.

Ein CNN besteht im Wesentlichen aus Filtern (Convolutional Layer) und Aggregations-Schichten (Pooling Layer), die sich abwechselnd wiederholen, und am Ende aus einer Schicht oder mehreren Schichten von "normalen" vollständig verbundenen Neuronen (Dense / Fully Connected Layer).

Details sind dem Stand der Technik zu entnehmen (siehe z.B.: S. Khan et al.: A Guide to Convolutional Neural Networks for Computer Vision, Morgan & Claypool Publishers 2018, ISBN 1681730227, 9781681730226).

Das Trainieren des neuronalen Netzes kann beispielsweise mittels eines Backpropagation-Verfahrens durchgeführt werden. Dabei wird für das Netz eine möglichst zuverlässige Abbildung von gegebenen Eingabevektoren auf gegebene Ausgabevektoren angestrebt. Die Qualität der Abbildung wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich der Beziehung der schwach- und der stark kontrastverstärkten MRT-Aufnahmen der dynamischen und/oder hepatobiliären Phase und MRT-Aufnahmen ohne Kontrastverstärkung, die verwendet werden können, um eine oder mehrere MRT-Aufnahmen vorherzusagen, die einen Untersuchungsbereich mit starker Kontrastverstärkung zeigen und die nun nur mittels MRT-Aufnahmen ohne Kontrastverstärkung und schwach kontrastverstärkten MRT-Aufnahmen berechnet werden können.

Eine Kreuzvalidierungsmethode kann verwendet werden, um die Daten in Trainings- und Validierungsdatensätze aufzuteilen. Der Trainingsdatensatz wird beim Backpropagations-Training der Netzwerkgewichte verwendet. Der Validierungsdatensatz wird verwendet, um zu überprüfen, mit welcher Vorhersagegenauigkeit sich das trainierte Netzwerk auf unbekannte Mehrzahlen von MRT-Aufnahmen anwenden lässt.

## Patentansprüche

1. Verfahren umfassend die Schritte
- Empfangen mindestens einer ersten MRT-Aufnahme eines Untersuchungsobjekts, wobei die mindestens eine erste MRT-Aufnahme eine Leber oder einen Teil einer Leber des Untersuchungsobjekts ohne eine durch ein Kontrastmittel herbeigeführte Kontrastverstärkung zeigt,
- Empfangen mindestens einer zweiten MRT-Aufnahme eines Untersuchungsobjekts, wobei die mindestens eine zweite MRT-Aufnahme eine Leber oder einen Teil einer Leber des Untersuchungsobjekts zeigt, wobei Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind,
- Zuführen von den empfangenen MRT-Aufnahmen an ein Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts ohne eine durch ein Kontrastmittel herbeigeführte Kontrastverstärkung zeigen und von MRT-Aufnahmen derselben Leber oder demselben Teil der Leber des gleichen Untersuchungsobjekts bei dem Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind, eine oder mehrere MRT-Aufnahmen vorherzusagen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- Erzeugen einer oder mehrerer vorhergesagter MRT-Aufnahmen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

2. Verfahren gemäß einem der vorherigen Ansprüche, wobei die mindestens eine zweite MRT-Aufnahme eine Leber oder einen Teil einer Leber des Untersuchungsobjekts zeigt, wobei Blutgefäße und/oder gesunde Leberzellen in der Leber, nach Applikation einer Dosis die höchstens 8% bevorzugt höchstens 5% der vollen Dosis eines hepatobiliären, paramagnetischen Kontrastmittels entspricht, kontrastverstärkt dargestellt sind, und das Vorhersagemodell mittels überwachten Lernens trainiert worden ist, anhand von MRT-Aufnahmen, die eine Leber oder einen Teil einer Leber eines Untersuchungsobjekts ohne eine durch ein Kontrastmittel herbeigeführte Kontrastverstärkung zeigen und von MRT-Aufnahmen derselben Leber oder demselben Teil der Leber des gleichen Untersuchungsobjekts bei dem Blutgefäße und/oder gesunde Leberzellen in der Leber, nach Applikation einer Dosis die höchstens 8% bevorzugt höchstens 5% der vollen Dosis eines hepatobiliären, paramagnetischen Kontrastmittels entspricht, kontrastverstärkt dargestellt sind, eine oder mehrere MRT-Aufnahmen vorherzusagen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann.

3. Verfahren gemäß einem der Ansprüche 1 und 2, wobei es sich bei der mindestens einen zweiten MRT-Aufnahme um eine T1-gewichtete Darstellung der Leber oder des Teils der Leber in der dynamischen Phase nach Applikation eines hepatobiliären, paramagnetischen Kontrastmittels handelt.

4. Verfahren gemäß Anspruch 3, wobei jeweils mindestens eine zweite MRT-Aufnahme die Leber oder einen Teil der Leber des Untersuchungsobjekts während der arteriellen Phase zeigen, verwendet wird.

5. Verfahren gemäß einem der Ansprüche 1 bis 3, wobei es sich bei dem Kontrastmittel um das Dinatriumsalz der Gadoxetsäure handelt.

6. Verfahren gemäß Anspruch 5, wobei 100% der Dosis des Dinatriumsalz der Gadoxetsäure 0,025 mmol/kg Körpergewicht bzw. 0,1 ml/kg Körpergewicht entspricht.

7. Verfahren gemäß einem der vorherigen Ansprüche, wobei beim Untersuchungsobjekt um ein Säugetier, vorzugsweise einen Menschen handelt.

8. Verfahren gemäß einem der vorherigen Ansprüche, wobei es sich bei dem Vorhersagemodell um ein künstliches neuronales Netz handelt.

9. System umfassend
• eine Empfangseinheit,
• eine Steuer- und Recheneinheit und
• eine Ausgabeeinheit,
- wobei die Steuer- und Recheneinheit konfiguriert ist, die Empfangseinheit zu veranlassen, mindestens eine erste MRT-Aufnahme eines Untersuchungsobjekts zu empfangen, wobei die mindestens eine erste MRT-Aufnahme eine Leber oder einen Teil einer Leber des Untersuchungsobjekts ohne eine durch ein Kontrastmittel herbeigeführte Kontrastverstärkung zeigt,
- wobei die Steuer- und Recheneinheit konfiguriert ist, die Empfangseinheit zu veranlassen, mindestens eine zweite MRT-Aufnahme eines Untersuchungsobjekts zu empfangen, wobei die mindestens eine zweite MRT-Aufnahme dieselbe Leber oder denselben Teil der Leber zeigt, wobei Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind,
- wobei die Steuer- und Recheneinheit konfiguriert ist, anhand der empfangenen MRT-Aufnahmen eine oder mehrere MRT-Aufnahmen vorherzusagen, wobei die eine oder die mehreren vorhergesagten MRT-Aufnahmen die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- wobei die Steuer- und Recheneinheit konfiguriert ist, die Ausgabeeinheit zu veranlassen, eine oder der mehrere vorhergesagte MRT-Aufnahmen anzuzeigen, auszugeben oder in einem Datenspeicher zu speichern.

10. Computerprogrammprodukt umfassend ein Computerprogramm, das in einen Arbeitsspeicher eines Computers geladen werden kann und dort den Computer dazu veranlasst, folgende Schritte ausführen:
- Empfangen mindestens einer ersten MRT-Aufnahme eines Untersuchungsobjekts, wobei die mindestens eine erste MRT-Aufnahme eine Leber oder einen Teil einer Leber des Untersuchungsobjekts ohne eine durch ein Kontrastmittel herbeigeführte Kontrastverstärkung zeigt,
- Empfangen mindestens einer zweiten MRT-Aufnahme desselben Untersuchungsobjekts, wobei die mindestens eine zweite MRT-Aufnahme eine Leber oder einen Teil einer Leber des Untersuchungsobjekts zeigt, wobei Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind,
- Zuführen von den empfangenen MRT-Aufnahmen an ein Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts ohne eine durch ein Kontrastmittel herbeigeführte Kontrastverstärkung zeigen und von MRT-Aufnahmen derselben Leber oder demselben Teil der Leber des gleichen Untersuchungsobjekts bei dem Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind, eine oder mehrere MRT-Aufnahmen vorherzusagen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- Erzeugen einer oder mehrerer vorhergesagter MRT-Aufnahmen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

11. Computerprogrammprodukt gemäß Anspruch 10, wobei das Computerprogramm, den Computer dazu veranlasst, einen oder mehrere der in den Ansprüchen 1 bis 7 aufgeführten Schritte auszuführen.

12. Verwendung eines hepatobiliären, paramagnetischen Kontrastmittels in einem MRT-Verfahren, wobei das MRT-Verfahren die folgenden Schritte umfasst:
- Erzeugen mindestens einer ersten MRT-Aufnahme, wobei die mindestens eine erste MRT-Aufnahme die Leber oder einen Teil der Leber ein Untersuchungsobjekts ohne eine durch ein Kontrastmittel herbeigeführte Kontrastverstärkung zeigt,
- Applizieren einer Dosis des hepatobiliären, paramagnetischen Kontrastmittels die höchstens 10% der vollen Dosis des hepatobiliären, paramagnetischen Kontrastmittels entspricht, wobei sich das Kontrastmittel in der Leber des Untersuchungsobjekts verteilt,
- Empfangen mindestens einer zweiten MRT-Aufnahme, wobei die mindestens eine zweite MRT-Aufnahme dieselbe Leber oder denselben Teil der Leber des Untersuchungsobjekts zeigt, wobei Blutgefäße und/oder gesunde Leberzellen in der Leber kontrastverstärkt dargestellt sind,
- Zuführen von den empfangenen MRT-Aufnahmen an ein Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts ohne eine durch ein Kontrastmittel herbeigeführte Kontrastverstärkung zeigen und von MRT-Aufnahmen derselben Leber oder demselben Teil der Leber des gleichen Untersuchungsobjekts bei dem Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind, eine oder mehrere MRT-Aufnahmen vorherzusagen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- Erzeugen einer oder mehrerer vorhergesagter MRT-Aufnahmen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

13. Hepatobiliäres, paramagnetisches Kontrastmittel zur Verwendung in einem MRT-Verfahren, wobei das MRT-Verfahren die folgenden Schritte umfasst:
- Erzeugen mindestens einer ersten MRT-Aufnahme, wobei die mindestens eine erste MRT-Aufnahme die Leber oder einen Teil der Leber ein Untersuchungsobjekts ohne eine durch ein Kontrastmittel herbeigeführte Kontrastverstärkung zeigt,
- Applizieren einer Dosis des hepatobiliären, paramagnetischen Kontrastmittels die höchstens 10% der vollen Dosis des hepatobiliären, paramagnetischen Kontrastmittels entspricht, wobei sich das Kontrastmittel in der Leber des Untersuchungsobjekts verteilt,
- Empfangen mindestens einer zweiten MRT-Aufnahme, wobei die mindestens eine zweite MRT-Aufnahme dieselbe Leber oder denselben Teil der Leber des Untersuchungsobjekts zeigt, wobei Blutgefäße und/oder gesunde Leberzellen in der Leber kontrastverstärkt dargestellt sind,
- Zuführen von den empfangenen MRT-Aufnahmen an ein Vorhersagemodell, wobei das Vorhersagemodell mittels überwachten Lernens trainiert worden ist, anhand von MRT-Aufnahmen, die eine Leber oder einen Teil einer Leber eines Untersuchungsobjekts ohne eine durch ein Kontrastmittel herbeigeführte Kontrastverstärkung zeigen und von MRT-Aufnahmen derselben Leber oder demselben Teil der Leber des gleichen Untersuchungsobjekts bei dem Blutgefäße und/oder gesunde Leberzellen in der Leber, nach Applikation einer Dosis die höchstens 10% der vollen Dosis eines hepatobiliären, paramagnetischen Kontrastmittels entspricht, kontrastverstärkt dargestellt sind, eine oder mehrere MRT-Aufnahmen vorherzusagen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- Erzeugen einer oder mehrerer vorhergesagter MRT-Aufnahmen, die die Leber oder einen Teil der Leber des Untersuchungsobjekts mit einer durch ein Kontrastmittel herbeigeführte Kontrastverstärkung zeigen, die durch Applikation einer Dosis die 100% der vollen Dosis des Kontrastmittels entspricht, herbeigeführt werden kann,
- Anzeigen und/oder Ausgeben der einen oder der mehreren vorhergesagten MRT-Aufnahmen und/oder Speichern der einen oder der mehreren vorhergesagten MRT-Aufnahmen in einem Datenspeicher.

14. Hepatobiliäres, paramagnetisches Kontrastmittel zur Verwendung gemäß Anspruch 13, wobei es sich bei dem Kontrastmittel um das Dinatriumsalz der Gadoxetsäure handelt und vorzugsweise 100% der Dosis des Dinatriumsalz der Gadoxetsäure 0,025 mmol/kg Körpergewicht bzw. 0,1 ml/kg Körpergewicht entspricht.

15. Kit umfassend ein Kontrastmittel gemäß Anspruch 13 und ein Computerprogrammprodukt gemäß Anspruch 9 oder 10.
